# EUROPEAN PATENT APPLICATION

(11) **EP 1 254 718 A2**
(43) Date of publication of application: **06.11.2002**
(21) Application number: 02009847.1
(22) Date of filing: 02.05.2002
(51) Int. Cl.: B01L 3/14, G01N 21/07, G01N 21/03, A61B 10/00

(54) **Evacuated tube and method for microscopy examination of urine sediment, chemistry and microbiological assays**

(30) Priority: 04.05.2001 US 288750 P
(71) Applicant: Becton Dickinson and Company, Franklin Lakes, New Jersey 07417-1880 (US)
(72) Inventor: Harrop, Andrew John, Eggbuckland, Plymouth, Devon PL6 5th (GB)
(74) Representative: Selting, Günther, Dipl.-Ing.

(57) **Abstract**

A urine specimen container assembly is provided. The assembly includes an evacuated tube and a closure for maintaining the evacuated condition in the tube and for sealing the tube after placement of a urine specimen therein. The tube includes an open top for engagement by the closure and a cylindrical wall extending from the open top. A conical base extends from the lower end of the cylindrical wall to the bottom of the tube. Fins extend outwardly from the conical base and define a profile that substantially replicates the profile of a round-bottom tube. Thus, the tube can be used in chemical analyzers configured for round bottom tubes and can be used in a centrifuge to enable accumulation of sediment in the conical base.

## Description

### BACKGROUND OF THE INVENTION

### 1. Field of the Invention

The present invention relates to a tube, and particularly a urine specimen tube, that enables efficient collection of a specimen, and that can be used with any of a plurality of different types of laboratory test apparatus.

### 2. Description of the Prior Art

A urine sample is conventionally collected by a patient in an open-topped receptacle, and then is transferred by a nurse or a medical technician into a container. The container then is closed and shipped to a laboratory for analysis. A technician at the laboratory will open the container and pour portions of the urine specimen into each of several other containers for analysis. For example, a portion of the urine specimen may be poured into a tube that has a conical base configured for use in a centrifuge. The tube is closed and centrifuged such that cells, casts or crystals concentrate in the conical base of the tube as sediment. Liquid portions of the specimen can be poured off, and the sediment retained in the conical portions of the tube can be transferred to a microscope slide for subsequent examination.

A portion of the urine specimen also may be presented to an automated chemistry analyzer. Analyzers for these purposes typically are adapted to receive a tube having a round or spherically generated base. Thus, a portion of the original urine specimen is poured from the original container into a conventional tube with a round or spherically generated base that is adaptable for use with chemistry analyzers.

Other portions of the urine specimen may be transferred directly to a slide for microscopic analysis. Still other portions may be accessed by a dipstick for analysis. The pouring of the urine specimen from its original cup or other such receptacle into the shipping container and the subsequent pouring into each of a plurality of differently configured tubes creates the potential for contaminating the specimen. Furthermore, the frequent pouring of urine specimens from one container to another both in a medical facility and in a laboratory and the corresponding opening and closing of containers creates the potential for contamination of personnel in the medical facility or laboratory. Additionally, the potential exists for container leakage during transport. Such leakage also presents the potential for contaminating the sample, contaminating other nearby samples or contaminating personnel who must remove the improperly sealed containers at the laboratory.

### SUMMARY OF THE INVENTION

The present invention is a tube for simplifying the transfer of urine specimens and for reducing the number of transfers required to properly test and analyze a urine specimen.

Desirably, the tube of the present invention significantly reduces contamination to either the urine specimen or to the user.

Preferably, the tube includes a closed bottom, an open top and a side wall extending between the top and bottom. The tube may be evacuated, and the open top of the tube may be sealed by a puncturable diaphragm. The interior of the tube may be accessed by a needle cannula that pierces through the puncturable diaphragm for depositing a sample of urine therein.

The transfer of the urine into the evacuated tube is facilitated by the low pressure conditions existing in the tube and the relatively higher pressure existing at locations externally of the tube. Thus, the urine specimen need not be poured carefully into the evacuated tube. Rather, the needle cannula that pierces the diaphragm may have an end connected to a straw which can be placed in communication with the urine specimen. Thus, a flow of the urine into the evacuated tube is achieved by the pressure differential across the diaphragm, namely, a low pressure within the evacuated tube and a higher pressure at the supply of urine externally of the tube.

Preferably, the tube may be unitarily molded from a plastic material and may include a substantially cylindrical side wall extending from the open top toward the bottom of the tube. The closed bottom end of the tube preferably defines a conical base. The conical base of the tube is oriented such that the major diameter end of the conical base is oriented upwardly and intersects or meets the cylindrical side wall of the tube. The bottom end of the tube is further characterized by a plurality of substantially identical circumferentially spaced fins. Each fin may be substantially planar and includes an outer edge. The outer edge includes a circular edge portion configured and disposed such that the circular edge of each fin and the cylindrical wall of the tube have locations that are tangent to a single imaginary plane. The circular portions on the outer edges of the respective fins meet at the extreme bottom end of the tube and have a point that is tangent to an imaginary plane aligned orthogonally to the tube. Thus, the outer edges of the respective fins collectively define a profile that resembles the outer profile of a conventional prior art tube with a round bottom.

The tube of the subject invention may be used in a centrifuge, as explained above and also may be used in an automated chemistry analyzer adapted for receiving a tube with a round or spherically generated bottom. Thus, a single tube can be used both for the chemical analysis and for the centrifugation. This reduces the number of tubes required for a urine specimen and further reduces the pouring or transfer steps required for the urine specimen.

### DESCRIPTION OF THE DRAWINGS

FIG. 1 is a perspective view of the tube of the present invention.

FIG. 2 is a side elevational view of the tube of FIG. 1.

FIG. 3 is a bottom plan view of the tube of FIG. 1.

FIG. 4 is a schematic illustration of the convention art method of collecting a urine specimen for analysis.

FIG. 5 is a schematic illustration of the method of the present invention.

### DETAILED DESCRIPTION

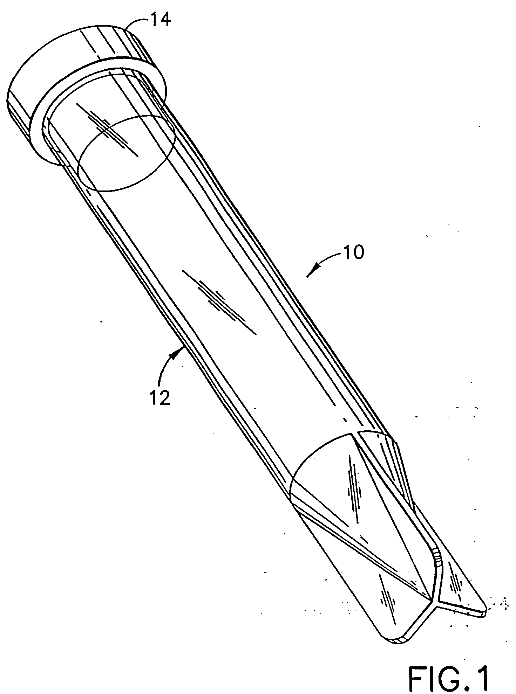

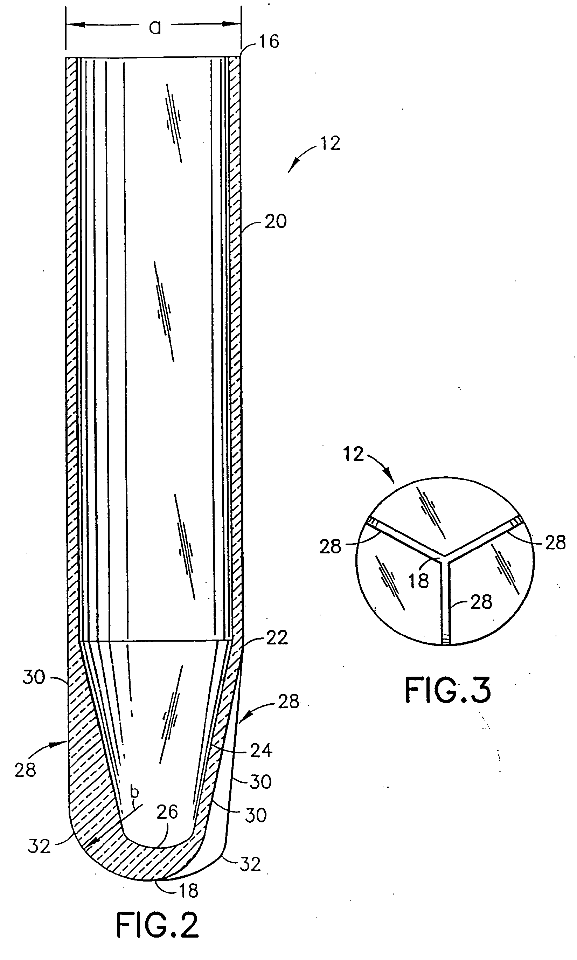

As shown in FIGS. 1 and 2, an assembly **10** comprises a tube **12** and a closure **14**. Tube **12** is unitarily molded from a thermoplastic material and includes an open top **16** and a closed bottom **18**. A substantially cylindrical side wall **20** extends from open top **16** to a location **22** which is between open top **16** and closed bottom **18** and defines an outside diameter "a".

Tube **12** further includes a substantially conical base **24** which extends from location **22** at the bottom of cylindrical side wall **20** to bottom end **18** of tube **12**. More particularly, conical base **24** has a top end which is unitary with cylindrical side wall **20** at location **22**, and hence defines substantially the same diameter. Conical base **24** tapers to smaller cross-sectional dimensions at locations further from cylindrical side wall **20**, and terminates in a small diameter spherically bottom wall **26**.

Tube **12** further includes fins **28** which extend unitarily outwardly from conical base **24**. As shown in FIG. 3, fins **28** are substantially identical to one another and are substantially equally spaced from one another about conical base **24** by angles of approximately **120**. Each fin **28** includes a straight edge **30** which extends colinearly from cylindrical side wall **20**. Each fin **28** further includes a circular edge **32** of radius "b" which equals approximately one-half outside diameter "a" of cylindrical wall **20.** Circular edge **32** of each fin **28** is tangent to straight edge **30** thereof. Furthermore, circular edges **32** of all three fins **28** are substantially tangent to an imaginary plane that is normal to the axis of tube **12** at bottom end **18**. Fins 28 define an external profile in proximity to bottom end **18** of tube **12** that substantially conforms to the external profile of a conventional tube having a round or spherically generated bottom. The combination of conical base **24** and fins **28** enable tube **12** to be used both for chemical analysis of the specimen and for centrifugation. This dual configuration reduces inventory requirements of tubes and reduces the manipulation and pouring of urine from the original container. As a result, the risk of contamination to either the specimen or to the user is reduced.

Closure **14** of assembly **10** comprises a stopper that is dimensioned to be sealingly engaged in open top **16** of tube **12**. At least central portions of stopper **14** are formed from an elastomeric material that is pierceable or puncturable by a needle cannula and resealable. Closure **14** may further include a hard thermoplastic shell that is engaged with elastomeric portions of closure **14** for facilitating manipulation as closure **14** is inserted in and/or removed from open top **16** of tube **12.**

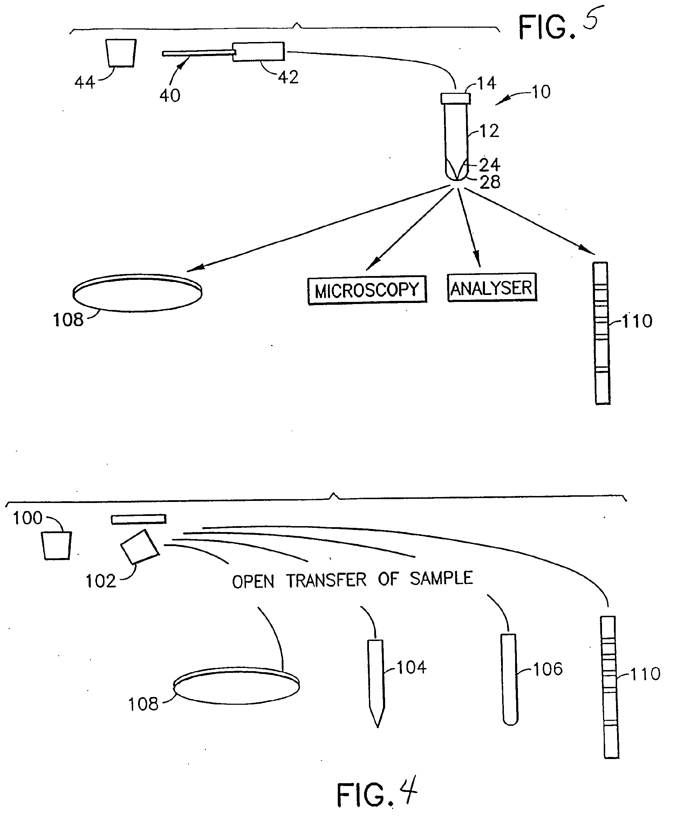

A urine specimen is collected conventionally as illustrated schematically in Fig. 4. A urine sample is collected by a patient in an open-top receptacle **100**, and then is transferred by a nurse or a medical technician into a container **102**. Container **102** then is closed and shipped to a laboratory for analysis. A technician at the laboratory will open container **102** and pour portions of the urine specimen into each of several other containers for analysis. For example, a portion of the urine specimen may be poured into a tube **104** that has a conical base configured for use in a centrifuge. Tube **104** is closed and centrifuged such that cells, casts or crystals concentrate in the conical base of tube **104** as sediment. Liquid portions of the specimen can be poured off, and the sediment retained in the conical portions of tube **104** can be transferred to a microscope slide for subsequent examination.

A portion of the urine specimen also may be presented to an automated chemistry analyzer. Chemistry analyzers typically are adapted to receive a tube having a round or spherically generated base. Thus, a portion of the original urine specimen is poured from container **102** into a tube **106** with a round or spherically generated base that is adaptable for use with chemistry analyzers.

Other portions of the urine specimen may be transferred directly to a slide **108** for microscopic analysis. Still other portions may be accessed by a dipstick **110** for analysis. The pouring of the urine specimen from its original cup or other such receptacle **100** into container **102** and the subsequent pouring into each of a plurality of differently configured tubes **104**, **106** creates the potential for contaminating the specimen. Furthermore, the frequent pouring of urine specimens from one container to another both in a medical facility and in a laboratory and the corresponding opening and closing of containers creates the potential for contamination of personnel in the medical facility or laboratory. Additionally, the potential exists for container leakage during transport. Such leakage also presents the potential for contaminating the sample, contaminating other nearby samples or contaminating the user who must remove the improperly sealed containers at the laboratory.

The present invention in use is shown in FIG. 5. A urine specimen is placed in assembly **10** by a hollow straw **40** having a metallic or hard plastic cannula **42** securely attached to one end, as shown in FIG. 5. Cannula **42** includes a lumen that communicates with the passage through hollow straw **40**. Cannula **42** further includes a pointed end that can be pierced through closure **14** of assembly **10**. Straw **40** and cannula **42** are employed by placing the end of the straw **40** remote from cannula **42** in communication with a urine specimen in a low cost cup **44** and by urging the cannula **42** through closure **14** and partly into evacuated tube **12.** The pressure differential between evacuated tube **12** and the surrounding environment in which cup **44** is maintained generates a flow of the urine specimen into tube **12**. Thus, the tasks shown in FIG. 4, which involve manipulation and pouring of the initial container can be substantially eliminated. Simultaneously, the method shown schematically in FIG. 5, reduces the risk of contamination to the specimen.

Cannula **42** can be removed after an appropriate volume of the urine specimen has been deposited in tube **12**. If necessary, further portions of the urine specimen may be deposited in a like manner to other assemblies **10** of evacuated tubes **12** and closures **14**. Closure **14** will reseal itself after removal of cannula **42**. Hence, assembly **10** can be transported to a laboratory for analysis without risk of spillage or contamination during transport.

Assembly **10** can be placed in a chemical analyzer adapted for roundbottomed tubes. Circular edges **32** of fins **28** will substantially replicate the profile of a prior art round bottom tube, and hence will enable assembly **10** to be properly received in a chemical analyzer for automated chemical analysis of the urine specimen in tube **12** of assembly **10**. Assembly **10** also can be placed in a centrifuge and rotated sufficiently for sediment to move to conical base **24** of tube **12** in response to the centrifugal load. Closure **14** then can be removed from tube **12** and liquid portions of the urine specimen may be poured off. Sediment remaining in conical base **24** of tube **12** then may be removed for microscopic analysis.

The method illustrated schematically in FIG. 5 enables the inventory of tubes required for performing necessary analysis of a urine specimen to be minimized substantially. Additionally, manipulation and pouring of the urine specimen can be substantially reduced, thereby achieving a corresponding reduction in the risk of contamination of either the specimen or personnel handling the specimen.

It is apparent that various changes can be made to the present invention without departing from the scope of the invention as defined by the appended claims. For example, in alternate embodiments, four or more fins may be provided. Additionally, the specific configuration of closure **14** can take many forms that ensure maintenance of the evacuated state prior to use, sealability after use and relatively easy removal to access of a specimen in the tube.

## Claims

1. A urine collection tube having an open top and a closed bottom, a cylindrical side wall extending from said open top toward said closed bottom, a substantially conical base extending from said cylindrical side wall to said bottom of said tube, a plurality of substantially identical fins extending outwardly from said conical base, each said fin including a circular outer edge having a bottom end tangent to said bottom of said tube and a top end aligned such that a tangent from said top end is substantially colinear with said cylindrical side wall of said tube, whereby said conical base defines a receptacle for accumulation of sediment separated from a specimen in a centrifuge, and whereby said fins define a round bottom profile for said tube.

2. The tube of Claim 1, wherein said tube is unitarily formed from a thermoplastic material.

3. The tube of Claim 1, wherein said plurality of fins comprise three fins.

4. The tube of Claim 1, wherein each of said fins includes a straight edge extending colinearly from said tubular side wall of said tube, said straight edge being tangent to said circular edge at said top end of said circular edge.

5. The tube of Claim 1, wherein a cylindrical side wall of the tube is generated about a longitudinal axis, said bottom ends of said circular edges of said fins being substantially tangent to an imaginary plane extending orthogonal to said longitudinal axis.

6. The tube of Claim 1, wherein said conical base has a large diameter end and a small diameter end, said large diameter end being adjacent to and unitary with said cylindrical side wall of said tube.

7. A method for collecting a urine specimen for analysis comprising the steps of:
providing an evacuated tube comprising an open top tube and a pierceable closure;
providing an open-topped receptacle with said urine specimen;
providing a straw having opposed first and second ends, said first end being configured for piercing said closure;
placing said second end of said straw in communication with said urine specimen; and
placing said first end of said straw through said closure and at least partly into said evacuated tube, whereby pressure differentials between interior portions of said tube and exterior portions of said tube generate a flow of said urine into said tube for analysis.

8. A method of Claim 7, further comprising the step of removing the first end of the straw from the closure and allowing said closure to reseal itself.

9. The method of Claim 8, wherein the tube includes a conical base, and wherein the method further includes the step of subjecting said tube and said urine therein to a centrifuge for separating sediment from said specimen and retaining said sediment in said conical base.

10. The method of Claim 9, wherein the tube further includes a plurality of fins extending outwardly from said conical base, each said fin having a circular edge such that said circular edges of said fins define a round profile for said tube, said method further comprising the step of placing said round profile of said tube in a chemical analyzer for automated analysis of said urine specimen in said tube.
